# EUROPÄISCHE PATENTSCHRIFT

(11) **EP 2 438 844 B1**
(45) Veröffentlichungstag und Bekanntmachung des Hinweises auf die Patenterteilung: **20.04.2016**
(21) Anmeldenummer: 11177168.9
(22) Anmeldetag: 10.08.2011
(51) Int. Cl.: A61B 1/008, A61B 1/055, A61B 1/00, A61B 1/005

(54) **Gelenkabschnitt eines Schafts für ein endoskopisches Instrument**
Articulated section of a shaft for an endoscopic instrument
Section articulée d'un arbre pour un instrument endoscopique

(30) Priorität: 13.08.2010 DE 102010034380
(43) Veröffentlichungstag der Anmeldung: 11.04.2012
(73) Patentinhaber: Karl Storz GmbH & Co. KG, 78532 Tuttlingen (DE)
(72) Erfinder: Blase, Bastian, 10439 Berlin (DE)

(56) Entgegenhaltungen:
- WO-A2-2007/102152
- US-A1- 2003 065 358
- US-A1- 2007 055 103
- US-A1- 2008 027 279

## Beschreibung

Die vorliegende Erfindung betrifft einen Gelenkabschnitt eines Schafts für ein endoskopisches Instrument insbesondere für ein Endoskop nach dem Oberbegriff von Anspruch 1 sowie ein endoskopisches Instrument insbesondere ein Endoskop mit einem solchen Gelenkabschnitt.

Endoskope werden heute für eine Vielzahl von Anwendungen in Medizin und Technik verwendet. Endoskope umfassen typischerweise einen starren oder flexiblen lang erstreckten Schaft, der zur Einführung in einen Hohlraum geeignet ist, und an dessen Spitze ein Endoskopobjektiv zur Erzeugung eines Bildes einer Szene in dem beobachteten Hohlraum angeordnet ist. Zur Weiterleitung des endoskopischen Bildes vom distalen (d. h. beobachterfernen) Ende des Endoskops zum proximalen (beobachternahen) Ende, d. h. zum Bedienteil, kann beispielsweise ein geordnetes Bündel von Lichtleitfasern oder, bei starren Endoskopen, ein System von Stablinsen innerhalb des Schafts vorgesehen sein; am proximalen Ende des Endoskops kann das endoskopische Bild über ein Okular direkt betrachtet oder von einem elektronischen Bildaufnehmer zur weiteren Verarbeitung und Anzeige aufgenommen werden. Ein elektronischer Bildaufnehmer, beispielsweise ein CCD-Chip, kann aber auch im Bereich des distalen Endes des Schafts, d. h. an der Endoskopspitze, angeordnet sein ("chip on the tip"); in diesem Fall verlaufen innerhalb des Schafts elektrische Versorgungs- und Datenleitungen des Bildaufnehmers. Da in der Regel in dem beobachteten Hohlraum nicht ausreichend Licht vorhanden ist, ist ferner innerhalb des Schafts ein Lichtleitsystem vorgesehen, um ausreichend Licht an das distale Ende des Endoskops zu transportieren, wo es zur Beleuchtung des Hohlraums genutzt wird. Ferner können Kanäle für endoskopische Arbeitsinstrumente sowie beispielsweise Spül- und Saugleitungen durch den Schaft zum distalen Ende des Endoskopschafts geführt sein. Ferner umfasst ein Endoskop typischerweise einen Bedienteil, der am proximalen Ende des Schafts angeordnet ist und üblicherweise als Handgriff ausgebildet ist. Der Bedienteil kann beispielsweise Bedienelemente zur Steuerung des Bildaufnehmers, eine endoskopische Kamera und/oder Anschlüsse für Versorgungs- und Datenleitungen, für eine externe Lichtquelle und/oder für Spül- und Saugpumpen aufweisen.

Je nach Anwendungszweck und Erreichbarkeit des zu beobachtenden Hohlraums kommen starre oder flexible Endoskope zum Einsatz. Dabei ist die Blickrichtung bei starren Endoskopen in der Regel durch die Ausbildung des Objektivs festgelegt. In einer Vielzahl von Anwendungen ist es jedoch wünschenswert, das distale Ende des Endoskopschafts, d. h. die Endoskopspitze, abwinkeln zu können, um nicht nur die Ausrichtung der Endoskopspitze, sondern auch die Blickrichtung einer in der Endoskopspitze angeordneten Optik verändern zu können. Bei flexiblen Endoskopen ist es bekannt, einen Teilbereich des Schafts, insbesondere den distalen Endbereich des Schafts, aktiv biegbar und somit die Endoskopspitze abwinkelbar auszubilden. Dadurch kann ein Bereich des Hohlraums betrachtet werden, der weit größer ist als der Öffnungswinkel der Beobachtungsoptik. Hierdurch werden insbesondere Bereiche im menschlichen Körper besser erschlossen, die mit einem starren Endoskop nicht oder nicht vollständig betrachtet werden können, beispielsweise komplex gestaltete Hohlorgane wie der Magen oder der Darmtrakt, aber auch der insufflierte Bauchraum während einer Laparoskopie.

Der distale Endbereich des Schafts eines flexiblen Endoskops mit abwinkelbarer Spitze besteht zumeist aus gelenkig verbundenen Ringelementen, welche die Stützstruktur des Schafts bilden und über Bowdenzüge, häufig Biegesteuerzüge genannt, bedient und gegeneinander gekippt werden. Um das Einführen in den Hohlraum zu erleichtern und das Eindringen von Substanzen zu verhindern, sind die Ringelemente von einer flexiblen Hülle aus einem Kunststoffmaterial umgeben. Im Inneren der Ringelemente verlaufen insbesondere Licht- und Bildleitkabel, Kanäle für Fluide oder endoskopische Arbeitsinstrumente. Die Biegesteuerzüge sind entlang der Außen- oder Innenseite der Ringelemente geführt. Derartige flexible Endoskope sind beispielsweise in US 6,270,453 B1, US 6,482,149 B1 oder DE 101 43 966 B4 offenbart.

Auch die Patentanmeldungen US 2003/0065358 A1 und US 2008/0027279 A1 zeigen Endoskop-Systeme, die technische Lösungen anschneiden, Endoskop-Bauteile abzuwinkeln. Bei den abwinkelbaren Bauteilen handelt es sich in US 2003/0065358 A1 um Maulteile, bei US 2008/0027279 A1 um distale Arme.

Es ist auch bekannt, den distalen Endbereich des Schafts mit Hilfe speziell geformter Biegeelemente biegbar auszugestalten, die ebenfalls durch Bowdenzüge ausgelenkt werden können, wie beispielsweise in US 6,749,560 B1 und in EP 1 927 312 A1 beschrieben. Aus US 5,885,207 A1, US 6,550,3193 B1 und US 6,860,849 B2 ist es bekannt, dass der Schaft eines flexiblen Endoskops eine mehrschichtige stützende Hülle aufweist, die aus verschiedenen Materialien mit unterschiedlichen Werkstoffkennwerten aufgebaut ist, die sich zusammen zu einer steuerbaren flexiblen Schafthülle ergänzen. Auch selbstrückstellende Federn können zur Ausgestaltung eines steuerbaren flexiblen Schaftendes genutzt werden, wobei die Ansteuerung gleichfalls über Drahtzüge erfolgt; derartige Endoskope werden beispielsweise in WO 2008/060075 A1 und EP 1 849 400 A1 offenbart.

Dabei ist der minimale Radius, den der biegsame bzw. gelenkige Abschnitt des Schafts einnehmen kann, durch das jeweilige Konstruktionsprinzip vorgegeben. Beispielsweise erlaubt ein mit aufeinander folgenden, jeweils gelenkig miteinander verbundenen Ringelementen ausgebildeten Gelenkabschnitt nur einen relativ großen Biegeradius, da die einzelnen Ringelemente jeweils nur einen geringen Kippwinkel zum nächsten Ringelement aufweisen. Wenn die gelenkige Verbindung zweier Elemente nur eine Kippung um eine Achse ermöglicht, ist es für eine räumliche Biegemöglichkeit erforderlich, Elemente mit abwechselnd gegeneinander verdrehten Kippachsen anzuordnen, so dass nur jedes zweite Element in eine jeweils gewünschte Richtung auslenkbar ist; hierdurch wird der mögliche minimale Biegeradius nochmals vergrößert. Der Nahbereich neben dem Schaftende ist daher mit einer in der Endoskopspitze angeordneten Optik nicht einzusehen.

Im Rahmen der vorliegenden Anmeldung wird unter endoskopisches Instrument eines verstanden, das zur Einführung in einen Hohlraum und zur Beobachtung innerhalb des Hohlraums und/oder zur Durchführung von Manipulationen innerhalb des Hohlraums geeignet ist, wobei nicht unbedingt die Möglichkeit der optischen Beobachtung vorhanden sein muss. Endoskope zur Einführung in einen Hohlraum und zur Beobachtung innerhalb des Hohlraums sind Teil der endoskopischen Instrumente im Sinne dieser vorliegenden Anmeldung.

Es ist die Aufgabe der vorliegenden Erfindung, einen Gelenkabschnitt eines Schafts für ein endoskopisches Instrument sowie ein endoskopisches Instrument insbesondere ein Endoskop anzugeben, mittels derer eine Beobachtung auch des Nahbereichs und/oder Manipulation auch in dem Nahbereich des Schafts bzw. des Schaftendes möglich ist.

Diese Aufgabe wird durch einen Gelenkabschnitt gemäß Anspruch 1 sowie durch ein endoskopisches Instrument insbesondere ein Endoskop gemäß Anspruch 15 gelöst.

Ein Gelenkabschnitt eines Schafts verbindet einen proximalen Schaftteil mit einem distalen Schaftteil, beispielsweise einer abwinkelbaren Spitze des endoskopischen Instruments insbesondere Endoskops. Erfindungsgemäß ist der distale Schaftteil über zwei unterschiedlich lange starre Gelenkstäbe mit dem proximalen Schaftteil verbunden, wobei die Gelenkstäbe jeweils über ein Gelenk am distalen und am proximalen Schaftteil, insbesondere am distalen Ende des proximalen Schaftteils und am proximalen Ende des distalen Schaftteils ansetzen. Die Gelenkstäbe können beispielsweise mit Nieten oder Schrauben an den jeweiligen Schaftteilen fixiert sein und können in radialer Richtung nach innen und außen abwinkelbar sein.

Die Gelenke sind insbesondere als einachsige Gelenke ausgebildet, deren Achsen parallel zueinander stehen und jeweils quer zu einer Längsachse des betreffenden Schaftteils angeordnet sind, d. h., die Gelenkstäbe sind relativ zum distalen Ende des proximalen Schaftteils um Achsen schwenkbar, die näherungsweise senkrecht zu einer Längsachse des proximalen Schaftteils bzw. dessen distalen Endbereichs gerichtet sind, und sind relativ zum proximalen Ende des distalen Schaftteils um Achsen schwenkbar, die näherungsweise senkrecht zu einer Längsachse des distalen Schaftteils bzw. dessen distalen Endbereichs gerichtet sind.

Beide Teile des Schafts, insbesondere deren jeweilige Enden, können jeweils zylinderförmig ausgebildet sein. Insbesondere kann der distale Schaftteil eine gestreckte Stellung einnehmen, in der dieser eine gerade Fortsetzung des distalen Endbereichs des proximalen Schaftteils bildet. Hierfür können das distale Ende des proximalen Teils des Schafts und/oder das proximale Ende des distalen Schaftteils schräg abgeschnitten sein. Beide Schaftteile können weitere Funktionselemente enthalten, etwa mechanische Elemente zur Steuerung der Abwinkelung sowie Licht-, Versorgungs- und Datenleitungen.

Dadurch, dass der distale Schaftteil mit zwei unterschiedlich langen starren schwenkbaren Gelenkstäben mit dem proximalen Schaftteil verbunden ist, ist der distale Schaftteil und damit insbesondere eine abwinkelbare Spitze des endoskopischen Instruments insbesondere des Endoskops derart steuerbar, dass auch eine Beobachtung des Nahbereichs des Endoskops bzw. des Endoskopschafts oder eine Manipulation in diesem Bereich möglich ist. Eine derartige Vierpunkt-Gelenkanordnung verbindet mit der Abwinkelungsbewegung einen seitlichen Versatz, wodurch der distale Schaftteil bzw. die Spitze bei der Abwinkelung entgegen der Blickrichtung einer im distalen Schaftteil bzw. in der Endoskopspitze angeordneten Optik verlagert werden kann. Hierdurch sind auch sehr nahe Bereiche neben dem Endoskopschaft betrachtbar. Entsprechendes gilt für ein Instrument zur Manipulation.

Gemäß einer bevorzugten Ausführungsform der Erfindung ist die Abwinkelung der Spitze über eine Schubstange steuerbar, die mit einem weiteren Gelenk an einem mit einem der Gelenkstäbe verbundenen Hebel bzw. Arm ansetzt. Das weitere Gelenk kann insbesondere ein einachsiges Gelenk sein. Die Schubstange ist insbesondere zur Übertragung von Schub- und Zugbewegungen ausgebildet und wird daher auch als Zugstange bezeichnet. Durch eine Zugbewegung bzw. eine Schubbewegung der Schubstange wird der Arm in proximaler bzw. distaler Richtung gekippt und der mit dem Arm verbundene Gelenkstab entsprechend geschwenkt. Aufgrund der Vierpunkt-Kinematik der abwinkelbaren Spitze folgt der zweite Gelenkstab der Bewegung des ersten, jedoch aufgrund der unterschiedlichen Längen der beiden Gelenkstäbe mit einem unterschiedlichen Kippwinkel. Die Schubstange kann beispielsweise abschnittsweise flexibel ausgebildet sein und ist in bevorzugter Weise durch den Schaft bis zu einem Bedienteil des endoskopischen Instruments insbesondere des Endoskops geführt. Am Bedienteil können Betätigungselemente und/oder Antriebe angeordnet sein zur Verschiebung der Schubstange innerhalb des Schafts und damit zur Steuerung der Abwinkelung des distalen Schaftteils bzw. der abwinkelbaren Spitze.

In besonders bevorzugter Weise ist der Arm derart mit dem einem der Gelenkstäbe verbunden, dass der Anlenkpunkt der Schubstange in einem mittleren oder einem bevorzugten Winkelbereich der Abwinkelung mit dem Anlenkpunkt des Gelenkstabs am proximalen Schaftteil einen maximalen Hebelarm bildet. Dabei kann der bevorzugte Winkelbereich beispielsweise durch einen bevorzugten Blickwinkel einer in der abwinkelbaren Spitze angeordneten Optik gegeben sein. Beispielsweise kann der Gelenkstab um ca. 90° schwenkbar und der Arm in einem Winkel von 45° an dem mit dem proximalen Schaftteil verbundenen Ende des Gelenkstabs angeordnet sein. Durch eine derartige Anordnung des Arms ist eine besonders feinfühlige Steuerung der Bewegung des distalen Schaftteils bzw. einer abwinkelbaren Spitze möglich.

Ferner ist es vorteilhaft, wenn der Arm am kürzeren der beiden unterschiedlich langen Gelenkstäbe angebracht ist, da dieser bei der Abwinkelung die größere Winkelbewegung ausführt und somit eine feinfühligere Steuerung möglich ist. Insbesondere ist der Arm am proximalen Ende des kürzeren Gelenkstabs derart angeordnet, dass er bei gestreckter Stellung des Gelenkabschnitts schräg in distale Richtung nach innen zeigt. Hierdurch kann eine innerhalb des Schafts geführte Schubstange in besonders einfacher Weise am Arm angreifen. Dabei kann der Arm flexibel ausgeführt sein, um eine Querbewegung des Ansatzpunkts der Schubstange auszugleichen.

Gemäß einer alternativen Ausführungsform der Erfindung ist die Abwinkelung des distalen Schaftteils über eine Schubstange steuerbar, mit deren distalem Ende eine Zahnstange verbunden ist, die in ein mit einem der Gelenkstäbe verbundenes Zahnrad oder einen Teil eines Zahnrads eingreift. Bevorzugt ist das Zahnrad am proximalen Ende des kürzeren der beiden Gelenkstäbe angeordnet, wobei der Mittelpunkt des Zahnrads mit dem Drehpunkt des kurzen Gelenkstabs zusammen fällt. Das Zahnrad ist insbesondere als Sektor eines Zahnrads ausgebildet. Die Zahnstange, die beispielsweise rund oder rechteckig ausgebildet sein kann, greift in das Zahnrad ein und kann dieses durch axiales Verschieben drehen, wodurch der mit dem Zahnrad verbundene kurze Gelenkstab geschwenkt wird. Diese Ausführungsform hat den Vorteil, dass bei der Abwinkelung keine Querbewegung des distalen Endes der Schubstange auftritt. Die durch den Schaft geführte Schubstange kann zumindest abschnittsweise flexibel ausgebildet sein.

Gemäß einer weiteren alternativen Ausführungsform der Erfindung ist die Abwinkelung der abwinkelbaren Spitze über eine drehbare Welle steuerbar, mit deren distalem Ende eine Gewindestange bzw. ein Gewindestab verbunden ist, der in ein mit einem der Gelenkstäbe verbundenes Zahnrad eingreift. Der Gelenkstab und das mit diesem verbundene Zahnrad bzw. der Zahnradausschnitt können ähnlich wie bei der vorgenannten Ausführungsform ausgebildet sein. Der Gelenkstab wird somit durch Rotation der drehbaren Welle wie bei einem Schneckengetriebe über das Zahnrad geschwenkt. Dabei kann der Eingriff des Gewindestabs in das Zahnrad auf einen kurzen Bereich, insbesondere den distalen Bereich des Gewindestabs, beschränkt bleiben. Auch die drehbare Welle kann flexibel ausgebildet sein. Diese Ausführungsform hat den Vorteil, dass durch entsprechende Wahl der Gewindesteigung eine besonders feinfühlige Steuerung der Abwinkelung möglich ist.

In bevorzugter Weise ist die drehbare Welle relativ zum proximalen Teil des Schafts axial verschiebbar gelagert. Hierdurch wird es ermöglicht, auch bei einem Versagen oder Blockieren der Rotation des Gewindestabs diesen ähnlich wie eine Zahnstange axial zu verschieben, um den distalen Schaftteil in eine gestreckte Stellung zu bringen, die zumindest das Herausführen des Schafts aus dem Hohlraum gestattet.

Gemäß einer Ausführungsform der Erfindung ist der kürzere der beiden Gelenkstäbe kürzer als einem Durchmesser des Schafts entspricht. Insbesondere ist der kurze Gelenkstab kürzer als der Durchmesser des proximalen Schaftteils in dessen distalen Endbereich und ebenso kürzer als der Durchmesser des distalen Schaftteils in dessen proximalen Endbereich. Dies hat den Vorteil, dass die Abwinkelung der Spitze des endoskopischen Instrumentes erfolgen kann, ohne dass die Gefahr eines Blockierens bei einer bestimmten Winkelstellung besteht.

Gemäß einer andere Ausführungsform der Erfindung sind beide Gelenkstäbe länger als ein Durchmesser des Schafts, insbesondere als der Durchmesser des distalen Endbereichs des proximalen Schaftteils und der Durchmesser des proximalen Endbereichs des distalen Schaftteils. Hierdurch ist ein Abwinkeln der Spitze auch weit über 90° hinaus möglich. Hierfür sind bevorzugt beide Stäbe über einen Verstellmechanismus betätigbar, der beispielsweise einen seitlich vom Gelenkstab abstehenden Arm oder ein Zahnrad umfasst, woran eine Schubstange über ein weiteres Gelenk bzw. über eine Zahnstange bzw. einen Gewindestab angreift. Beide Gelenkstäbe werden dabei jeweils abwechselnd angesteuert. Bei kleinen Abwinkelungen wird der kurze Gelenkstab angesteuert, ab einer bestimmten Winkelstellung übernimmt jedoch der lange Stab die Steuerung der Abwinkelung, bevor der kurze Stab aufgrund der kinematischen Zusammenhänge blockieren würde.

Um zu verhindern, dass der nach innen kippende kurze Stab auf dem langen Stab aufsetzt, kann mindestens einer der Stäbe in Längsrichtung des Stabs geteilt sein, beispielsweise in ähnlicher Form wie eine Stimmgabel. Hierdurch können beide Stäbe sich ineinander verschränken, so dass eine weiter gehende Abwinkelung der Spitze des endoskopischen Instrumentes möglich ist.

Gemäß einer weiteren bevorzugten Ausführungsform der Erfindung setzen die beiden Gelenkstäbe in einander gegenüberliegenden Randbereichen des jeweiligen Schaftteils an. Insbesondere können der distale Endbereich des proximalen Schaftteils und der proximale Endbereich des distalen Schaftteils jeweils einen näherungsweise zylindrischen Außenschaft aufweisen, an dem die Gelenkstäbe jeweils nahezu gegenüberliegend angelenkt sind.

Hierdurch wird ein besonders günstiges Hebelverhältnis und damit eine hohe Stabilität der Anordnung aus den Enden der beiden Schaftteile und den beiden Gelenkstäben erzielt, andererseits steht der Innenraum der jeweiligen Schaftteile ohne wesentliche Einschränkungen für den Mechanismus zur Steuerung der Abwinkelung sowie ggf. für weitere Elemente zur Verfügung.

In vorteilhafter Weise verlaufen zwischen dem proximalen und dem distalen Schaftteil flexible Leitungen, die eine Verbindung von im distalen Schaftteil, insbesondere in der Spitze des endoskopischen Instrumentes insbesondere der Endoskopspitze, angeordneten optischen und elektronischen Bauelementen mit dem proximalen Schaftteil und weiter mit dem Bedienteil des endoskopischen Instrumentes insbesondere des Endoskops bzw. mit daran anschließbaren Geräten ermöglichen. Insbesondere ist in der abwinkelbaren Spitze eine Videoeinheit mit optischen Elementen wie Linsen sowie beispielsweise einem CCD-Chip als Bildaufnehmer angeordnet. Das digitale Bildsignal wird in diesem Fall über ein Datenleitkabel zum proximalen Ende des Endoskops zur weiteren Auswertung bzw. Betrachtung durch den Anwender geführt. Es könnte aber auch ein Endoskopobjektiv mit einer Bildleitung über flexible Glasfaserstränge, die gegen Ortsverschiebung gesichert sind, in der Endoskopspitze angeordnet sein. Zur Beleuchtung des zu betrachtenden Hohlraums ist in der Regel eine Beleuchtungsoptik mit Beleuchtungslichtleitern vorgesehen, die zur Endoskopspitze geführt sind. Ferner können weitere Versorgungs- und/oder Datenleitungen sowie beispielsweise Kanäle zum Spülen oder Saugen bzw. für flexible endoskopische Arbeitsinstrumente zwischen dem proximalen und dem distalen Schaftteil als flexible Leitungen geführt sein.

Weiterhin ist es bevorzugt, dass im Randbereich des distalen Endes des proximalen Schaftteils, insbesondere am distalen Ende eines Außenschafts des proximalen Schaftteils, Aussparungen angeordnet sind, in die sich die flexiblen Leitungen bei Abwinkelung des distalen Schaftteils hineinlegen. Bei einer Abwinkelung kann der distale Schaftteil bei einer Ausbildung am Beispiel eines Endoskops derart verlagert werden, dass dessen distales Ende, das bevorzugt das Fenster des Endoskopobjektivs und die Austrittsöffnungen der Beleuchtungslichtleiter bzw. der Instrumenten- und weiteren Kanäle aufweist, nahezu in Verlängerung einer Außenkontur des distalen Endes des proximalen Schaftteils zu liegen kommt. Der bei Geradeausstellung proximale Bereich des distalen Schaftteils bzw. der Endoskopspitze wird in diesem Fall entgegengesetzt verlagert und kann über die verlängerte Außenkontur des distalen Endes des proximalen Teils des Endoskopschafts hinaustreten. Auch die flexiblen Leitungen, die zwischen dem Schaft und der abwinkelbaren Spitze verlaufen, können daher über die Außenkontur hinaustreten. Um diese Leitungen, die einen gewissen Biegeradius aufweisen, in den proximalen Schaftteil zu führen, sind daher am Randbereich des distalen Endes des proximalen Schaftteils eine oder mehrere Aussparungen vorgesehen, in die sich die Leitungen bei einer Abwinkelung des distalen Schaftteils hineinlegen können. Der Biegeradius der Leitungen wird dadurch bei einer Abwinkelung gewissermaßen auf die der Blickrichtung und dem zu betrachtenden Bereich entgegengesetzte Seite verlagert. Daher schränkt der Biegeradius die Abwinkelung der Endoskopspitze nicht ein, so dass eine Abwinkelung um einen großen Winkel möglich ist und Bereiche unmittelbar neben dem Endoskopschaft beobachtbar sind.

In bevorzugter Weise sind die flexiblen Leitungen von einer oder mehreren abgedichteten flexiblen Hüllen umgeben, die einen Schutz der Leitungen gegen mechanische Beschädigungen sowie gegen das Eindringen von Verschmutzungen oder Flüssigkeiten bei der Benutzung des endoskopischen Instrumentes insbesondere des Endoskops sowie gegen das Eindringen von Reinigungs- und Desinfektionsfluiden bzw. Dampf beim Reinigen und Sterilisieren bieten. Insbesondere kann jede der Leitungen durch eine separate flexible Hülle umgeben sein, oder mehrere oder alle Leitungen können von einer flexiblen Hülle umgeben sein. Die flexible Hülle kann beispielsweise als bevorzugt metallischer Faltenbalg ausgebildet sein. Auch die Gelenkstäbe und/oder die Arm bzw. weitere Elemente des Verstellmechanismus können mit einer oder mehreren flexiblen Hüllen, beispielsweise Bälgen, abgedichtet sein. Durch geeignete Ausführung der flexiblen Hüllen kann es erreicht werden, dass das endoskopische Instrumentes oder Endoskop autoklavierbar ist.

Gemäß einer besonders bevorzugten Ausführungsform der Erfindung sind der distale und der proximale Teil des Schaft jeweils separat nach außen abgedichtet, insbesondere flüssigkeits- und dampfdicht abgedichtet. Hierdurch entstehen zwei separate, nach außen abgedichtete Bereiche. Die Leitungen, die zwischen dem distalen und dem proximalen Schaftteil geführt sind sowie ggf. der Betätigungsmechanismus für die Abwinkelung bzw. die Gelenkstäbe selbst können durch flexible Hüllen geschützt sein, die wiederum abgedichtet mit den beiden abgedichteten Bereichen verbunden sind. Hierdurch wird ein umfassender Schutz gegen das Eindringen von Substanzen und gegen mechanische Beschädigungen erzielt sowie eine weitere Verbesserung der Autoklavierfestigkeit.

In vorteilhafter Weise ist an der proximalen Seite des distalen Schaftteils und/oder am distalen Ende des proximalen Schaftteils eine Abdichtungsebene vorgesehen, die vom jeweiligen Endbereich nach innen, d. h., vom proximalen Ende des distalen Schaftteils nach distal bzw. vom distalen Ende des proximalen Schaftteils nach proximal versetzt ist. Hierdurch wird auch bei einer Abwinkelung des distalen Schaftteils um einen großen Winkel stets ein ausreichend großer Biegeradius der flexiblen Leitungen ermöglicht sowie ein ausreichender Bewegungsraum für die Gelenkstäbe bereitgestellt. Die Dichtebenen können schräg im jeweiligen Schaftteil bzw. in der Spitze des endoskopischen Instrumentes liegen oder senkrecht zur jeweiligen Längsachse angeordnet sein. Sie sind durch eine dampfdichte Verbindung, beispielsweise durch Anschweißen, mit einer Innenseite eines jeweiligen Außenschafts verbunden.

Ein erfindungsgemäßes endoskopisches Instrument insbesondere ein Endoskop weist einen zur Einführung in einen Hohlraum ausgebildeten Schaft insbesondere Endoskopschaft und einen Bedienteil auf. Der Schaft weist einen erfindungsgemäßen Gelenkabschnitt auf zur Abwinkelung eines distalen Schaftteils, beispielsweise einer steuerbaren Endoskopspitze, gegenüber einem proximalen Schaftteil. Der Bedienteil kann Bedienelemente aufweisen, beispielsweise zur Betätigung der Abwinkelung des distalen Schaftteils durch eine Verschiebung der Schubstange bzw. eine Rotation der drehbaren Welle. Ferner kann der Bedienteil Anschlüsse aufweisen, beispielsweise für eine Kameraeinheit, für Saug- und Spülpumpen und/oder für endoskopische Arbeitsinstrumente. Der Bedienteil ist insbesondere als Handgriff ausgebildet.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 eine Rückansicht eines Gelenkabschnitts gemäß einem ersten Ausführungsbeispiel der Erfindung;
Fig. 2 eine isometrische Ansicht des Gelenkabschnitts gemäß dem ersten Ausführungsbeispiel;
Fig. 3 eine Seitenschnittansicht durch den Gelenkabschnitt gemäß dem ersten Ausführungsbeispiel;
Fig. 4 eine isometrische Schnittansicht in der Biegeebene des Gelenkabschnitts gemäß dem ersten Ausführungsbeispiel;
Fig. 5 eine isometrische Gesamtansicht des Gelenkabschnitts gemäß einem zweiten Ausführungsbeispiel der Erfindung;
Fig. 6 eine Seitenschnittansicht entlang der Biegeebene des Gelenkabschnitts gemäß dem zweiten Ausführungsbeispiel;
Fig. 7 eine Seitenschnittansicht des Gelenkabschnitts gemäß einem dritten Ausführungsbeispiel der Erfindung;
Fig. 8 eine isometrische Ansicht des Gelenkabschnitts des dritten Ausführungsbeispiels;
Fig. 9 eine Seitenschnittansicht des Gelenkabschnitts gemäß einem vierten Ausführungsbeispiel;
Fig. 10 eine isometrische Ansicht des Gelenkabschnitts des vierten Ausführungsbeispiels;
Fig. 11 eine Seitenschnittansicht des Gelenkabschnitts gemäß einem fünften Ausführungsbeispiel der Erfindung;
Fig. 12 eine isometrische Ansicht des Gelenkabschnitts des fünften Ausführungsbeispiels;
Fig. 13 eine schematische isometrische Darstellung des Bewegungsablaufs bei einem erfindungsgemäßen Gelenkabschnitt;
Fig. 14 schematische Seitenschnittansichten zur Verdeutlichung des Bewegungsablaufs bei einem Gelenkabschnitt gemäß einer weiteren Ausführungsform der Erfindung.

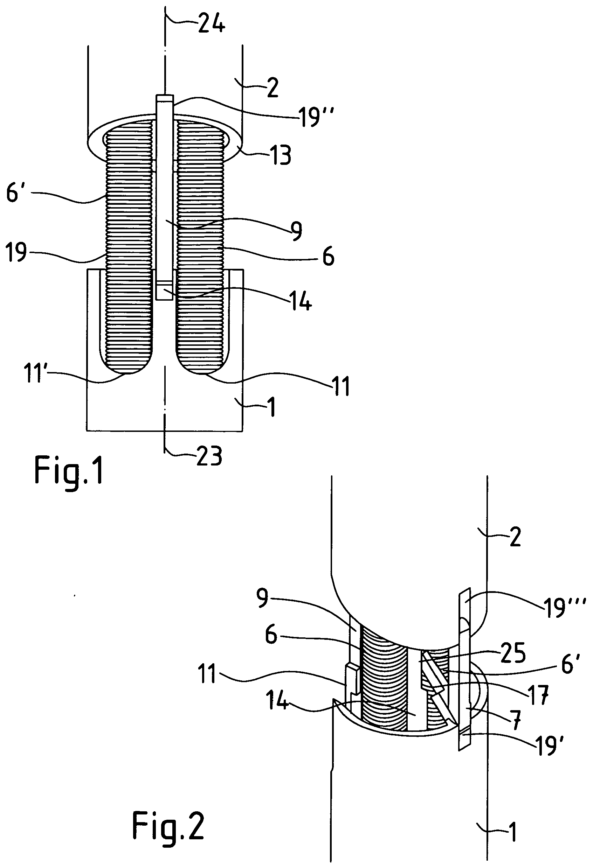

Wie in Fig. 1 und 2 gezeigt, weist ein Gelenkabschnitt eines Schaftes eines endoskopischen Instrumentes inbesondere eines Endoskops gemäß einer ersten Ausführungsform der Erfindung zwei Gelenkstäbe 7, 9 auf, die eine gelenkige Verbindung zwischen einem proximalen Teil 1 und einem distalen Teil 2 des Schafts herstellen. In den Figuren 1 bis 12 sind jeweils der distale Endbereich des proximalen Schaftteils 1 und der proximalen Endbereich des distalen Schaftteils 2 dargestellt. Der proximale Schaftteil 1 kann mit seinem proximalen Ende mit einem Bedienteil verbunden sein (nicht dargestellt). Der distale Schaftteil 2 kann die Spitze des endoskopischen Instrumentes sein oder mit dieser verbunden sein. In der Spitze des endoskopischen Instrumentes insbesondere des Endoskops befinden sich eine Videoeinheit sowie die Ausgänge der weiteren Kanäle (nicht dargestellt).

Wie in Fig. 1 gezeigt, sind im Außenschaft des proximalen Schaftteils 1 zwei Nuten 19, 19' vorgesehen, die das Einpassen der Gelenkstäbe 7, 9 erlauben und weit genug ausgeführt sind, um das Zusammenfalten des Gelenks während der Abwinkelung nicht zu behindern. In den Nuten 19, 19' sind die Gelenkstäbe jeweils um eine zur Längsachse 23 des distalen Endes des proximalen Schaftteils 1 senkrechte Achse schwenkbar. Entsprechend ist in einem zylindrischen Außenrohr des distalen Schaftteils 2 eine Nut 19" zur Aufnahme des distalen Endes des Gelenkstabs 9 vorhanden, worin dieser um eine zur Längsachse 24 des proximalen Endes des distalen Schaftteils 2 senkrechte, zur Schwenkachse im proximalen Schaftteil parallele Achse schwenkbar gelagert ist. Eine entsprechende Nut zur Aufnahme des anderen Gelenkstabs 7 und zur Festlegung einer ebenfalls hierzu parallelen Schwenkachse ist in der Ansicht der Fig. 1 verdeckt.

Gemäß Fig. 1 und 2 verlaufen zwischen den beiden Schaftteilen 1, 2 Versorgungs-, Daten- und Lichtleitungen sowie weitere Kanäle, die durch zwei Wellenbälge bzw. Faltenbälge 6, 6' geschützt sind. Die Bälge 6, 6' sind relativ zur Ebene der Gelenkstäbe einander gegenüberliegend angeordnet, wobei zwischen den Bälgen der Auslenkarm 17 und die Schubstange 14 verlaufen. Wie insbesondere in Fig. 1 erkennbar, weist der zylindrische Außenschaft des proximalen Schaftteils 1 auf seiner rückwärtigen, d. h. in abgewinkelter Stellung einer Blickrichtung der in der Endoskopspitze angeordneten Optik entgegengesetzten Seite zwei Aussparungen 11, 11' auf, in die sich die beiden Faltenbälge 6, 6' beim Auslenken des distalen Schaftteils 2 einschmiegen können, um während des Abwinkelungsvorgangs nicht geknickt oder zerdrückt zu werden. In Fig. 1 ist nur der dorsale Gelenkstab 9 erkennbar. Der distale Schaftteil 2 weist an seinem proximalen Ende eine schräge Abschlussfläche 13 auf.

In Fig. 2 ist eine isometrische Gesamtansicht des Gelenkbereichs dargestellt. Der frontale Gelenkstab 7 weist einen Hebel bzw. Auslenkarm 17 auf, an welchem eine ansteuerbare Schubstange 14 angebracht ist, um über den Arm 17 den frontalen Gelenkstab 7 und damit das ganze Gelenk auszulenken. In Fig. 2 sind auch die frontalen und dorsalen Nuten 19, 19' des als Endoskopschafts 1 ausgebildeten Schaft des endoskopischen Instrumentes sowie die frontale Nut 19'" der als Endoskopspitze 2 ausgebildeten Spitze des endoskopischen Instrumentes zu erkennen. Die Schubstange 14 greift über ein Drehgelenk 25 an dem Auslenkarm 17 des kürzeren frontalen Gelenkstabs 7 an. Um eine Einschränkung des Schwenkwinkelbereichs zu vermeiden, weisen die Schubstange 14 und der Auslenkarm 17 wie in Fig. 2 dargestellt im Bereich des Drehgelenks 25 jeweils Abflachungen bzw. Aussparungen auf.

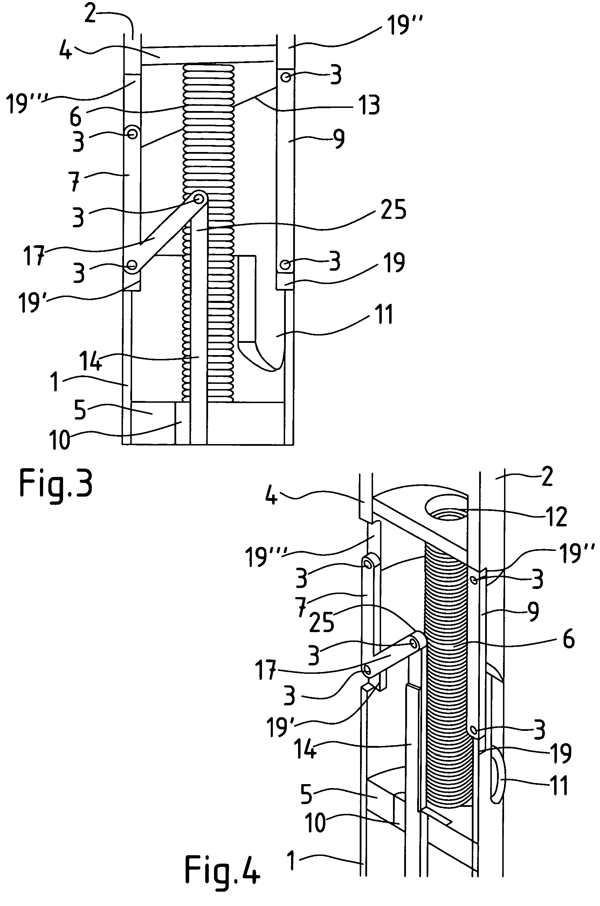

In dem in Fig. 1 und Fig. 2 dargestellten gestreckten, d. h. nicht abgewinkelten Zustand liegt der distale Schaftteil 2 in gerader Verlängerung des proximalen Schaftteils 1, so dass die jeweiligen Längsachsen 23, 24 zusammenfallen und die jeweiligen Außenkonturen auf einer gemeinsamen Zylinderfläche liegen. Durch Ziehen an der Schubstange 14 aus der in Fig. 1 und Fig. 2 dargestellten Position in proximaler Richtung kann der distale Schaftteil nach frontal abgewinkelt werden. Auch in den Figuren 3 bis 12 ist jeweils der gestreckte Zustand gezeigt.

In Fig. 3 ist zur Verdeutlichung des Gelenkaufbaus und der Ansteuerung mit der Schubstange eine Seitenschnittansicht des Gelenks gemäß der ersten Ausführungsform dargestellt, wobei gemäß Fig. 3 und Fig. 4 Maßnahmen zur Abdichtung getroffen sein können. Der distale Teil 1 und der proximale Teil 2 des Schafts sind demgemäß jeweils an ihrem proximalen bzw. distalen Ende durch Dichtebenen 4, 5 gegen das Eindringen von Substanzen versiegelt. Über den Wellenbalg 6 sind beide Bereiche miteinander verbunden. Hierfür ist der Wellenbalg 6 abgedichtet in die Dichtebenen 4, 5 eingesetzt. Der frontale, kurze Gelenkstab 7 und der dorsale, lange Gelenkstab 9 sind jeweils mit Nieten 3 drehbar am proximalen Schaftteil 1 und am distalen Schaftteil 2 angebracht. Auch die Schubstange 14 und der Auslenkarm 17, welcher unter 45° zum frontalen Gelenkstab 7 steht, sind über eine Niete 3 drehbar miteinander verbunden. Die Schubstange 14 ist flexibel ausgeführt, um eine leichte Querbewegung des Drehgelenks 25 während der axialen Verschiebung mitmachen zu können. Eine Dichtung 10 in der proximalen Dichtebene 5, die eine Verschiebung der Schubstange 14 zulässt, verhindert ein Eindringen von Substanzen in den proximalen Schaftteil 1. Die Dichtebenen 4, 5 sind von dem jeweiligen Endbereich des betreffenden Schaftteils nach innen, d. h. im proximalen Schaftteil 1 nach proximal und im distalen Schaftteil 2 nach distal versetzt, um genügend Bewegungsraum für den Arm 17, die Schubstange 14 und die Bälge 6, 6' zu bieten. In Fig. 3 ist auch die Abschrägung 13 des proximalen Endes des distalen Schaftteils 2 deutlich zu erkennen.

In der isometrischen Schnittansicht in Fig. 4, die die Raumverhältnisse im Gelenkabschnitt verdeutlicht, erkennt man zusätzlich die Öffnung 12 in der distalen Dichtebene 4, durch die Leitungen verlaufen können. Der Balg 6 ist flüssigkeits- und dampfdicht in die Öffnung 12 eingesetzt. In der proximalen Dichtebene 5 ist eine entsprechende Öffnung vorgesehen, in die der Balg 6 ebenfalls flüssigkeits- und dampfdicht eingesetzt ist.

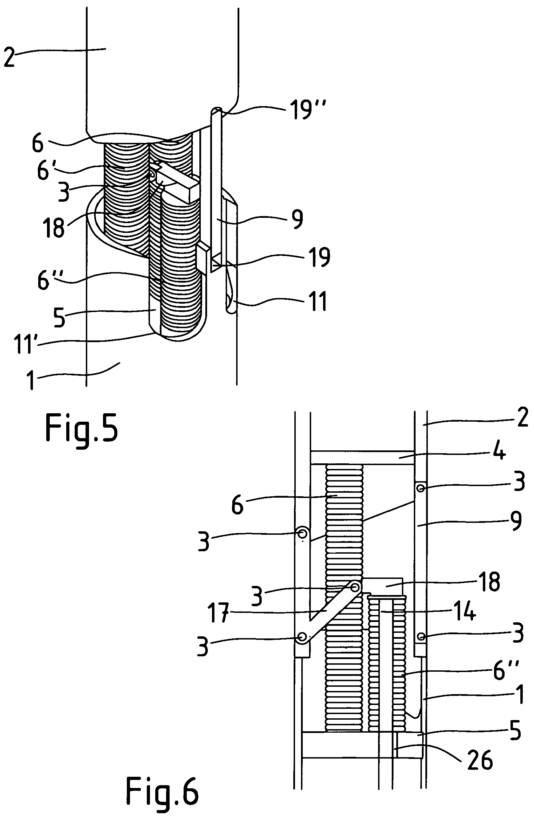

Um ein Eindringen von Dampf während des Autoklavierens in den Schaft insbesondere in den Endoskopschaft 1 zu verhindern, ist in der in Fig. 5 und Fig. 6 dargestellten Variante ein weiterer Balg 6" über die Schubstange 14 gesteckt. Da die Bälge 6, 6', 6" dampfdicht an den Dichtebenen 4, 5 angebracht sind, bleiben diese Übergänge sicher gegen Dampfeintritt. Der Balg 6" der Schubstange 14 schließt mit einem Aufsatzstück 18 ab, auf dessen unterer Seite die Schubstange 14 fixiert ist und dessen oberer Teil seitlich mit dem Auslenkarm 17 des frontalen Gelenkstabs 7 über eine Niete 3 gelenkig verbunden ist. Eine Dichtung wie in Fig. 3 und Fig. 4 innerhalb der proximalen Dichtebene 5 ist in diesem Fall aufgrund der Dichtheit des Balgs 6" nicht nötig. Für den Durchgang der Schubstange 14 durch die proximale Dichtebene 5 kann daher, wie in Fig. 6 gezeigt, eine größere Durchgangsöffnung 26 vorhanden sein, die der Schubstange 14 seitliches Spiel ermöglicht. Dadurch ist eine geringere Flexibilität der Schubstange 14 ausreichend, um mit dem Balg 6" die Querbewegung während der Auslenkungsbewegung auszugleichen. Aus räumlichen Gründen sind in dieser Ausführungsform die beiden Bälge 6, 6' zur Durchleitung der Leitungen leicht nach frontal versetzt, um genügend Platz für den nach dorsal versetzten dritten Balg 6" über der Schubstange 14 zu gewährleisten. Dabei ist ausreichend Abstand vorhanden, so dass die Bälge 6, 6', 6" sich bei der Abwinkelungsbewegung nicht gegenseitig berühren oder behindern und die Bälge 6, 6' sich in die Aussparungen 11, 11' einfügen können.

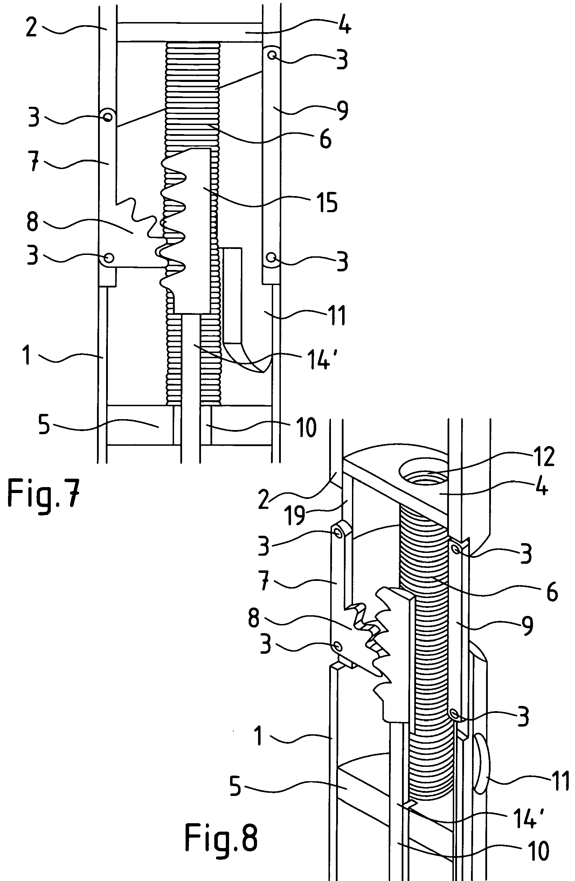

In Fig. 7 und 8 ist eine dritte Ausführungsform der Erfindung dargestellt. Gemäß Fig. 7 und 8 weist der frontale Gelenkstab 7 anstelle eines Auslenkarms einen Zahnradausschnitt 8 auf, dessen Mittelpunkt mit dem Drehpunkt des Stabs 7 zusammenfällt. In den Zahnradausschnitt 8 greift eine Zahnstange 15 ein, die mit dem distalen Ende einer Schubstange 14' verbunden ist; die Schubstange 14' kann mit der Zahnstange 15 einstückig ausgebildet sein. Die Schubstange 14' verläuft durch eine Dichtung 10 in der proximalen Dichtebene 5. Eine Querbewegung findet bei dieser Ausführungsform nicht statt, so dass die Schubstange 14' in diesem Bereich keine Flexibilität aufweisen muss.

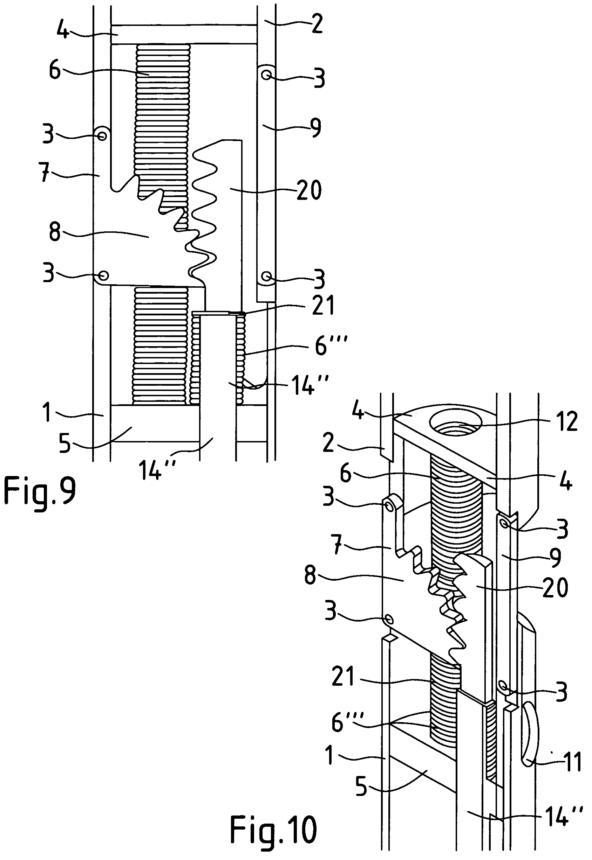

Um die Dampfdichtheit beim Autoklavieren zu verbessern, kann gemäß der in Fig. 9 und 10 dargestellten vierten Ausführungsform der Erfindung über einer Schubstange 14" ein weiterer Balg 6"' auf der proximalen Dichtebene 5 aufgebracht sein. Dieser schließt dampfdicht mit einer Deckelplatte 21 ab, an deren unterer Seite die Schubstange 14" sitzt und auf deren oberer Seite eine kurze Zahnstange 20 fixiert ist. Auch bei dieser Variante muss die Schubstange 14" nicht die gleiche Flexibilität wie die Schubstange der ersten Ausführungsform aufweisen, da hier keine Querauslenkung zu erwarten ist. Außerdem ist keine zusätzliche Dichtung in der proximalen Dichtebene 5 nötig, da der Balg 6'" für eine ausreichende Dichtheit sorgt.

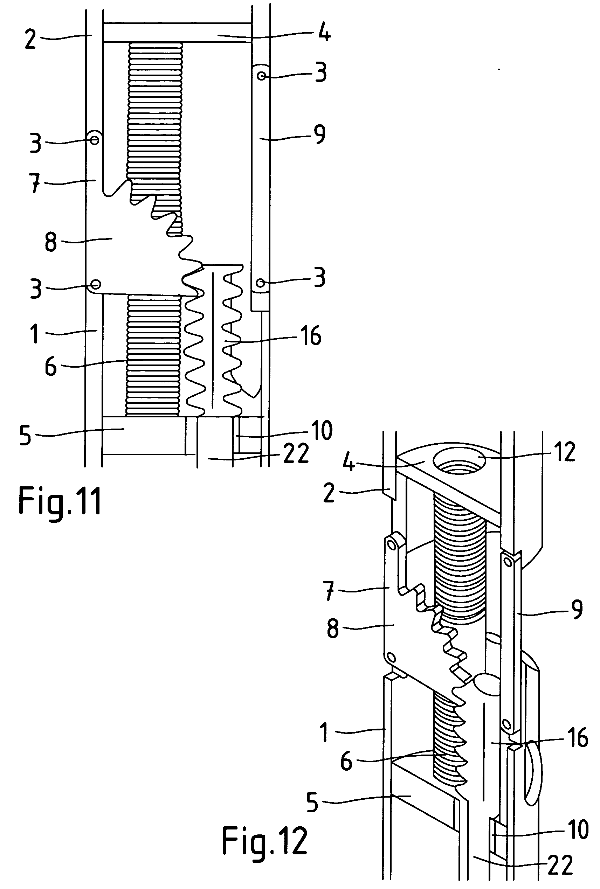

Eine fünfte Ausführungsform der Erfindung ist in Fig. 11 und 12 dargestellt. Der angesteuerte frontale Gelenkstab 7 weist auch hier einen Zahnradausschnitt 8 auf, der beispielsweise größer als bei der dritten und vierten Ausführungsform ausgeführt sein kann, und über eine Gewindestange 16 wie in einem Schneckengetriebe bewegt wird. Gemäß dieser Variante erfolgt die Abwinkelung des Gelenks über eine Rotation der Gewindestange 16, die an einer durch den Schaft geführten drehbaren Welle 22 angebracht ist. Diese verläuft durch eine Dichtung 10 in der proximalen Dichtebene 5, um ein Eindringen von Substanzen in den Schaft, der hier als Endoskopschaft 1 ausgebildet ist, zu vermeiden.

Aus Platzgründen sind die beiden Bälge 6, 6' und die Gewindestange 16 nicht in einer Linie, sondern versetzt angeordnet, wobei ein ausreichender Abstand besteht, so dass die Bälge 6, 6' beim Auslenken an der Gewindestange 16 vorbei in die Aussparungen 11, 11' passen. Der Gewindebereich der Gewindestange 16 muss nur wenige Gänge umfassen, da immer nur wenige Zähne des Zahnradsektors 8 eingreifen.

Für den Fehlerfall, dass die Rotation der Gewindestange 16 versagt, kann es vorgesehen sein, dass sich diese trotzdem noch wie die Zahnstange 15 axial verschieben lässt, um die Spitze 2 wieder in die gestreckte Stellung zu bringen. Hierfür ist es vorteilhaft, dass die Gewindestange 16 mehr Gänge umfasst als für den Betrieb durch Rotation der Gewindestange 16 erforderlich wären.

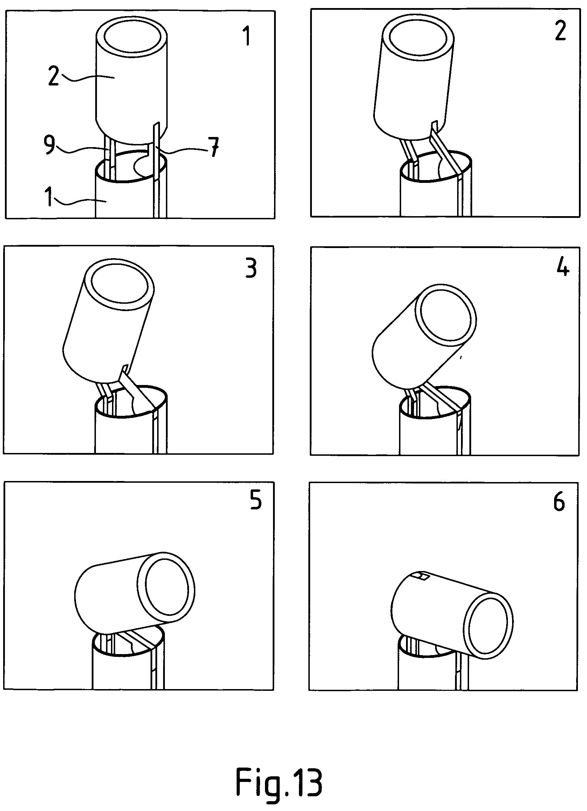

Wie in Fig. 13 für eine bis etwa 90° abwinkelbare als Endoskopspitze ausgebildete Spitze des endoskopischen Instrumentes schematisch dargestellt, bewegt sich der kurze Stab 7 zur Abwinkelung des distalen Schaftteils 2, der hier die Endoskopspitze darstellt, aus der gestreckten Stellung (Fig. 13, Teilbild 1) um seinen proximalen Gelenkpunkt radial nach innen. Auf Grund der Kopplung der einzelnen Elemente verlagert sich die Endoskopspitze dabei nach dorsal (Teilbild 2). Mit zunehmender Kippbewegung des kurzen Stabs 7 nach innen geht die Verschiebung der Spitze in eine Drehung über (Teilbilder 3 bis 5). In der Endstellung ist die Spitze um ca. 90° abgewinkelt und nach hinten, d. h. nach dorsal verlagert (in Teilbild 6 aus dorsaler Richtung gesehen). Das polyzentrische Gelenk hat sich somit während der Bewegung zusammen gefaltet.

Die in Fig. 13 aus Gründen der Übersichtlichkeit nicht dargestellten Faltenbälge passen sich der Bewegung an und folgen der Endoskopspitze. Sie biegen sich dorsal hinter dem Endoskop aus. Der unvermeidliche Biegeradius der Licht- und Bildleitkabel wird daher gewissermaßen nach hinten verschoben. Damit die Faltenbälge dabei von den beiden Schaftteilen nicht zusammengedrückt und abgeknickt werden, sind am distalen Ende des proximalen Schaftteils Aussparungen angebracht, in die sich die Bälge während der Abwinkelung legen können (s. Fig. 1 bis 12). Zwischen beiden Aussparungen bleibt etwas Material stehen, um den hinteren, längeren Stab am proximalen Schaftteil anbringen zu können. Auf Grund ihrer federnden Eigenschaften wirken die beiden Bälge sowohl rückstellend als auch versteifend gegen Querbiegung. Das Ausmaß ihrer Ausdehnung nach hinten und die rückstellende Kraft während der Abwinkelung hängen u. a. von ihrer Länge ab. Werden die Dichtebenen gemäß Fig. 3 bis 13 etwas nach innen in den Schaft verlagert, können die Bälge auf größerer Länge die Biegung besser verteilen, so dass die Auskragungen nach hinten und der benötigte Kraftaufwand geringer sind.

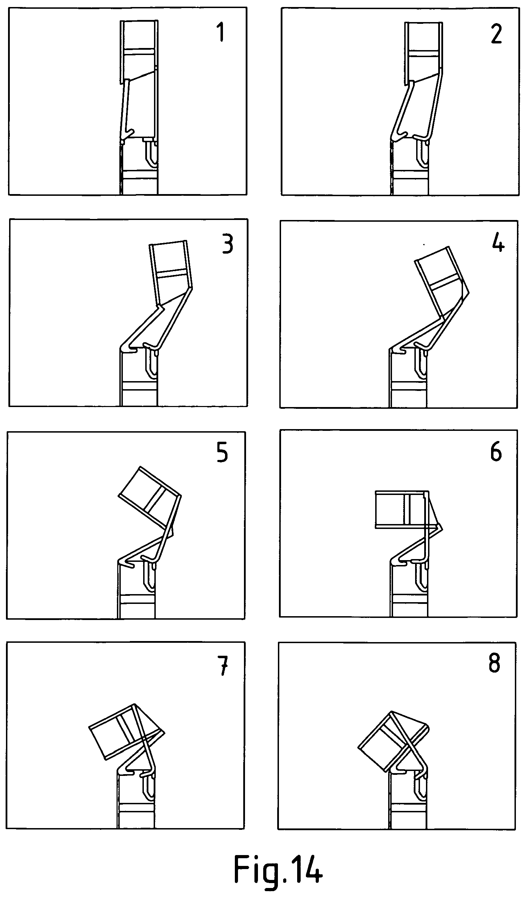

Ein Abwinkeln der Spitze bzw. der Endoskopspitze auch weit über 90° hinaus ist möglich, wenn beide Gelenkstäbe der Vierpunkt-Polyzentrik länger als der Schaftdurchmesser sind (s. Fig. 14). Dazu weist der längere Gelenkstab einen ähnlichen Arm bzw. einen Zahnradsektor wie der kürzere Gelenkstab auf und wird in ähnlicher Weise wie der kürzere Gelenkstab über eine Schubstange bzw. eine drehbare Welle angesteuert (in Fig. 14 nicht dargestellt). Dabei wird zu jedem Zeitpunkt nur jeweils einer der beiden Gelenkstäbe über die entsprechende Schubstange bzw. drehbare Welle betätigt. Auch hierbei geht, ausgehend von der gestreckten Stellung (Fig. 14, Teilbild 1) die Verlagerung der Spitze bzw. der Endoskopspitze (Teilbild 2) in eine Kippbewegung über (Teilbilder 3 bis 5). In der in Fig. 14 gezeigten Anordnung ist die Bewegung nicht auf eine Abwinkelung bis zu 90° (Teilbild 6) beschränkt, sondern geht darüber hinaus (Teilbild 7) und kann zu einer unmittelbar dem distalen Ende des proximalen Schaftteils benachbarten Position der distalen Endfläche der Spitze bzw. Endoskopspitze führen (Teilbild 8). Dabei übernimmt ab einer bestimmten Winkelstellung der Betätigungsmechanismus des langen Stabs die Abwinkelung, bevor der kurze Stab aus kinematischen Gründen blockieren würde. Um zu verhindern, dass der nach innen kippende kurze Stab auf dem langen Stab aufsetzt, ist einer der Stäbe, im gezeigten Beispiel der lange Stab, ähnlich einer Stimmgabel teilweise geteilt. Hierdurch können sich beide Gelenkstäbe ineinander verschränken und die Spitze bzw. die Endoskopspitze weiter abwinkeln. Auch bei dieser Ausführungsform können die Betätigungselemente mit Bälgen abgedichtet werden.

In den Figuren sind gleiche oder ähnliche Teile jeweils mit denselben Bezugszeichen versehen. Der Übersichtlichkeit halber sind nicht alle Bezugszeichen in allen Figuren eingetragen.

### Bezugszeichenliste

- 1: Proximaler Schaftteil
- 2: Distaler Schaftteil
- 3: Niete
- 4: Dichtebene
- 5: Dichtebene
- 6,6',6",6'": Balg
- 7: Kurzer Gelenkstab
- 8: Zahnrad
- 9: Langer Gelenkstab
- 10: Dichtung
- 11, 11': Aussparung
- 12: Öffnung
- 13: Abschrägung
- 14, 14', 14": Schubstange
- 15: Zahnstange
- 16: Gewindestab
- 17: Auslenkarm
- 18: Aufsatzstück
- 19, 19', 19", 19"': Nut
- 20: Zahnstange
- 21: Deckelplatte
- 22: Welle
- 23: Längsachse
- 24: Längsachse
- 25: Drehgelenk
- 26: Öffnung

## Patentansprüche

1. Gelenkabschnitt eines Schafts für ein endoskopisches Instrument zur abwinkelbaren Verbindung eines distalen Teils des Schafts mit einem proximalen Teil des Schafts, **dadurch gekennzeichnet, dass** der distale Schaftteil (2) über zwei unterschiedlich lange starre Gelenkstäbe (7, 9) mit dem proximalen Schaftteil (1) verbunden ist, die jeweils mit einem Gelenk am distalen und am proximalen Schaftteil ansetzen.

2. Gelenkabschnitt nach Anspruch 1, **dadurch gekennzeichnet, dass** eine Abwinkelung des distalen Teils des Schafts gegenüber dem proximalen Teil des Schafts über eine Schubstange (14) steuerbar ist, die über ein weiteres Gelenk (25) an einem mit einem der Gelenkstäbe (7, 9) verbundenen Arm (17) angreift.

3. Gelenkabschnitt nach Anspruch 1, **dadurch gekennzeichnet, dass** eine Abwinkelung des distalen Teils des Schafts gegenüber dem proximalen Teil des Schafts über eine Schubstange (14',14") steuerbar ist, an deren distalem Ende eine Zahnstange (15) angeordnet ist, die in ein mit einem der Gelenkstäbe (7, 9) verbundenes Zahnrad (8) eingreift.

4. Gelenkabschnitt nach Anspruch 1, **dadurch gekennzeichnet, dass** eine Abwinkelung des distalen Teils des Schafts gegenüber dem proximalen Teil des Schafts über eine drehbare Welle (22) steuerbar ist, an deren distalem Ende ein Gewindestab (16) angeordnet ist, der in ein mit einem der Gelenkstäbe (7, 9) verbundenes Zahnrad (8) eingreift.

5. Gelenkabschnitt nach Anspruch 4, **dadurch gekennzeichnet, dass** die drehbare Welle (22) relativ zum proximalen Teil des Schafts axial verschiebbar ist.

6. Gelenkabschnitt nach einem der Ansprüche 1 bis 5, **dadurch gekennzeichnet, dass** der kürzere (7) der beiden Gelenkstäbe kürzer ist als ein Durchmesser des Schafts.

7. Gelenkabschnitt nach einem der Ansprüche 1 bis 5, **dadurch gekennzeichnet, dass** beide Gelenkstäbe (7, 9) länger sind als ein Durchmesser des Schafts.

8. Gelenkabschnitt nach Anspruch 7, **dadurch gekennzeichnet, dass** zumindest einer der Gelenkstäbe (7, 9) geteilt ist.

9. Gelenkabschnitt nach einem der vorhergehenden Ansprüche, **dadurch gekennzeichnet, dass** die beiden Gelenkstäbe (7, 9) in einander gegenüberliegenden Randbereichen des proximalen und/oder des distalen Schaftteils (1, 2) ansetzen.

10. Gelenkabschnitt nach einem der vorhergehenden Ansprüche, **dadurch gekennzeichnet, dass** zwischen dem proximalen und dem distalen Schaftteil (1, 2) flexible Leitungen verlaufen.

11. Gelenkabschnitt nach Anspruch 10, **dadurch gekennzeichnet, dass** im Randbereich des distalen Endes des proximalen Schaftteils (1) Aussparungen (11, 11') angeordnet sind, in die sich die flexiblen Leitungen bei Abwinkelung des distalen Schaftteils (2) hineinlegen.

12. Gelenkabschnitt nach Anspruch 10 oder 11, **dadurch gekennzeichnet, dass** die flexiblen Leitungen von einer oder mehreren abgedichteten flexiblen Hüllen umgeben sind.

13. Gelenkabschnitt nach einem der vorhergehenden Ansprüche, **dadurch gekennzeichnet, dass** der proximale und der distale Schaftteil (1, 2) jeweils separat abgedichtet sind.

14. Gelenkabschnitt nach dem vorhergehenden Anspruch, **dadurch gekennzeichnet, dass** am proximalen und am distalen Schaftteil (1, 2) jeweils eine Abdichtungsebene (5, 4) vorgesehen ist, die von einem distalen Endbereich des proximalen Schaftteils (1) bzw. von einem proximalen Endbereich des distalen Schaftteils (2) nach proximal bzw. distal versetzt ist.

15. Endoskopisches Instrument, insbesondere Endoskop, mit einem Gelenkabschnitt gemäß einem der vorhergehenden Ansprüche sowie mit einem Bedienabschnitt, der Bedienelemente und/oder Anschlüsse aufweist.

## Claims

1. Hinge portion of a shank for an endoscopic instrument, for pivotably connecting a distal part of the shank to a proximal part of the shank, **characterized in that** the distal shank part (2) is connected to the proximal shank part (1) via two rigid hinge bars (7, 9) of different lengths, which each attach with a hinge to the distal and proximal shank parts.

2. Hinge portion according to Claim 1, **characterized in that** an angling of the distal part of the shank with respect to the proximal part of the shank can be controlled via a push rod (14), which engages via a further hinge (25) on an arm (17) connected to one of the hinge bars (7, 9).

3. Hinge portion according to Claim 1, **characterized in that** an angling of the distal part of the shank with respect to the proximal part of the shank can be controlled via a push rod (14', 14") on whose distal end a toothed rod (15) is arranged that engages in a toothed wheel (8) connected to one of the hinge bars (7, 9).

4. Hinge portion according to Claim 1, **characterized in that** an angling of the distal part of the shank with respect to the proximal part of the shank can be controlled via a rotatable shaft (22) on whose distal end a threaded bar (16) is arranged that engages in a toothed wheel (8) connected to one of the hinge bars (7, 9).

5. Hinge portion according to Claim 4, **characterized in that** the rotatable shaft (22) is axially movable relative to the proximal part of the shank.

6. Hinge portion according to one of Claims 1 to 5, **characterized in that** the shorter (7) of the two hinge bars is shorter than a diameter of the shank.

7. Hinge portion according to one of Claims 1 to 5, **characterized in that** both hinge bars (7, 9) are longer than a diameter of the shank.

8. Hinge portion according to Claim 7, **characterized in that** at least one of the hinge bars (7, 9) is divided.

9. Hinge portion according to one of the preceding claims, **characterized in that** the two hinge bars (7, 9) attach in mutually opposite edge areas of the proximal and/or distal shank part (1, 2).

10. Hinge portion according to one of the preceding claims, **characterized in that** flexible lines run between the proximal and distal shank parts (1, 2).

11. Hinge portion according to Claim 10, **characterized in that**, in the edge area of the distal end of the proximal shank part (1), recesses (11, 11') are arranged into which the flexible lines move during angling of the distal shank part (2).

12. Hinge portion according to Claim 10 or 11, **characterized in that** the flexible lines are enclosed by one or more sealed flexible sleeves.

13. Hinge portion according to one of the preceding claims, **characterized in that** the proximal and distal shank parts (1, 2) are each sealed separately.

14. Hinge portion according to the preceding claim, **characterized in that**, on the proximal and distal shank parts (1, 2), a sealing plane (5, 4) is in each case provided that is offset in the proximal or distal direction, respectively, from a distal end area of the proximal shank part (1) or from a proximal end area of the distal shank part (2).

15. Endoscopic instrument, in particular an endoscope, with a hinge portion according to one of the preceding claims, and with an operating portion, which has operating elements and/or connections.

## Revendications

1. Section articulée d'un arbre pour instrument endoscopique pour la connexion pouvant être pliée d'une partie distale de l'arbre à une partie proximale de l'arbre, **caractérisée en ce que** la partie distale de l'arbre (2) est connectée à la partie proximale de l'arbre (1) par le biais de deux barres d'articulation rigides de longueurs différentes (7, 9), qui sont montées à chaque fois avec une articulation à la partie distale et à la partie proximale de l'arbre.

2. Section articulée selon la revendication 1, **caractérisée en ce qu'**un coude de la partie distale de l'arbre peut être commandé par rapport à la partie proximale de l'arbre par le biais d'une tige de poussée (14) qui vient en prise par le biais d'une autre articulation (25) avec un bras (17) connecté à l'une des barres d'articulation (7, 9).

3. Section articulée selon la revendication 1, **caractérisée en ce qu'**un coude de la partie distale de l'arbre peut être commandé par rapport à la partie proximale de l'arbre par le biais d'une tige de poussée (14', 14") à l'extrémité distale de laquelle est disposée une crémaillère (15) qui s'engage dans une roue dentée (8) connectée à l'une des barres d'articulation (7, 9).

4. Section articulée selon la revendication 1, **caractérisée en ce qu'**un coude de la partie distale de l'arbre peut être commandé par rapport à la partie proximale de l'arbre par le biais d'un arbre rotatif (22) à l'extrémité distale duquel est disposée une bague filetée (16) qui s'engage dans une roue dentée (8) connectée à l'une des barres d'articulation (7, 9).

5. Section articulée selon la revendication 4, **caractérisée en ce que** l'arbre rotatif (22) peut être déplacé axialement par rapport à la partie proximale de l'arbre.

6. Section articulée selon l'une quelconque des revendications 1 à 5, **caractérisée en ce que** la plus courte (7) des deux barres d'articulation est plus courte qu'un diamètre de la tige.

7. Section articulée selon l'une quelconque des revendications 1 à 5, **caractérisée en ce que** les deux barres d'articulation (7, 9) sont plus longues qu'un diamètre de l'arbre.

8. Section articulée selon la revendication 7, **caractérisée en ce qu'**au moins l'une des barres d'articulation (7, 9) est divisée.

9. Section articulée selon l'une quelconque des revendications précédentes, **caractérisée en ce que** les deux barres d'articulation (7, 9) sont montées dans des régions de bord mutuellement opposées de la partie proximale et/ou de la partie distale (1, 2) de l'arbre.

10. Section articulée selon l'une quelconque des revendications précédentes, **caractérisée en ce que** des lignes flexibles s'étendent entre la partie proximale et la partie distale (1, 2) de l'arbre.

11. Section articulée selon la revendication 10, **caractérisée en ce que** des évidements (11, 11') sont réalisés dans la région de bord de l'extrémité distale de la partie proximale de l'arbre (1), dans lesquels s'insèrent les lignes flexibles lors du pliage de la partie distale de l'arbre (2).

12. Section articulée selon la revendication 10 ou 11, **caractérisée en ce que** les lignes flexibles sont entourées par une ou plusieurs douilles flexibles étanchéifiées.

13. Section articulée selon l'une quelconque des revendications précédentes, **caractérisée en ce que** la partie proximale et la partie distale (1, 2) de l'arbre sont chacune étanchéifiées séparément.

14. Section articulée selon l'une quelconque des revendications précédentes, **caractérisée en ce qu'**un plan d'étanchéité (5, 4) est prévu à chaque fois au niveau de la partie proximale et de la partie distale (1, 2) de l'arbre, lequel est décalé d'une région d'extrémité distale de la partie proximale de l'arbre (1) ou d'une région d'extrémité proximale de la partie distale de l'arbre (2) dans la direction proximale, respectivement distale.

15. Instrument endoscopique, en particulier endoscope, comprenant une section articulée selon l'une quelconque des revendications précédentes et comprenant une section de commande qui présente des éléments de commande et/ou des raccords.
